# FASCICULE DE BREVET EUROPEEN

(11) **EP 0 651 990 B1**
(45) Date de publication et mention de la délivrance du brevet: **14.10.1998**
(21) Numéro de dépôt: 94402359.7
(22) Date de dépôt: 20.10.1994
(51) Int. Cl.: A61K 7/02, A61K 7/48

(54) **Composition à base d'esters en C12 à C20 et utilisation dans le démaquillage**
Zusammensetzung auf der Basis von C12-C20 Estern und Verwendung zum Abschminken
Composition based on C12-C20 esters for make-up removal

(30) Priorité: 05.11.1993 FR 9313162
(43) Date de publication de la demande: 10.05.1995
(73) Titulaire: L'OREAL, 75008 Paris (FR)
(72) Inventeur: Marion, Catherine, F-92330 Sceaux (FR); Louvet, Nathalie, F-94150 Rungis (FR); Le Fleche, Marie-Josée, F-91380 Chilly Mazarin (FR); Lukassen, Liliane, F-94150 Chevilly Larue (FR)
(74) Mandataire: Lhoste, Catherine

(56) Documents cités:
- EP-A- 0 370 856
- EP-A- 0 422 862
- US-A- 4 810 489

## Description

La présente invention se rapporte à une composition formée d'une phase aqueuse et d'une phase grasse comprenant un ester, ainsi qu'à son utilisation dans le soin démaquillant du visage y compris des yeux.

Cette composition peut se présenter sous la forme d'une lotion biphasique, d'un lait, d'une crème ou encore d'un gel. Elle convient au démaquillage de tout type de peaux, allant notamment des peaux sensibles aux peaux à tendance grasse.

On connaît, selon le document JP-A-55-150402, des démaquillants contenant des esters dont le nombre d'atomes de carbone varie de 17 à 36 ("esters en C₁₇ à C₃₆") et dans une proportion d'au moins 20 % en poids.

D'après ce document, pour obtenir un effet démaquillant, il est indispensable de respecter cette teneur d'au moins 20% en poids d'ester, et pour éviter un effet irritant, il faut que cet ester soit au moins en C₁₇. Pour cette dernière raison, les auteurs de ce document préfèrent les esters en C₂₃ au moins.

D'autre part, pour la préparation de démaquillants liquides, ils indiquent que l'on peut incorporer jusqu'à 30% en poids d'esters en C₁₇ à C₂₂.

Dans le cas de démaquillants solides, ils précisent que l'on peut associer aux 20% ou plus d'esters, des agents solidifiants de type paraffineux à raison de 5% à 80% en poids, ainsi que d'autres additifs huileux tels que des hydrocarbures.

Ainsi les démaquillants illustrés dans le document ci-dessus, formés d'esters en C₁₇ à C₃₆, de préférence C₂₃ à C₂₈, possèdent tous des teneurs très élevées en corps gras, dans tous les cas supérieures à 20%.

Pour cette raison, ces démaquillants peuvent provoquer, au moment de leur application, un désagrément ou inconfort qui se traduit par une sensation de lourdeur sur le visage ou de voile sur les yeux. Par ailleurs, en raison de leur texture "lourde", ces démaquillants manquent de fraîcheur, sont difficiles à "travailler" et leur rinçage n'est pas aisé.

Après l'application, un rinçage à l'aide d'un tonique ou d'eau s'avère en fait indispensable.

Par ailleurs, en raison des fortes concentrations en corps gras dans ce type de démaquillants, se posent des problèmes d'odeur comme c'est le cas avec les esters en C₂₂. Il est alors nécessaire de masquer l'odeur par un parfumage intense, ce qui peut provoquer des picotements ou d'autres problèmes de tolérance.

On constate donc que subsiste dans l'art antérieur le besoin de compositions démaquillantes efficaces, d'emploi simple - c'est-à-dire sans rinçage obligatoire - à faibles teneurs en gras et ne présentant pas un manque de confort pendant et après l'application sur le visage et/ou les yeux et présentant une totale innocuité.

Or, à la suite d'importantes recherches menées sur la question, la demanderesse a maintenant découvert qu'il est possible de remédier aux inconvénients des compositions de l'art antérieur en utilisant une composition particulière comprenant en faibles quantités une certaine famille d'esters convenablement sélectionnée.

La présente invention a ainsi pour objet une composition formée d'une phase aqueuse et d'une phase grasse, ladite phase grasse comprenant au moins un ester, caractérisée en ce que ledit ester a un nombre total d'atomes de carbone allant de 12 à 20, en ce qu'il est présent à une teneur pondérale comprise entre 0,1% et 10% du poids total de la composition et en ce que la phase grasse représente moins de 20 % en poids par rapport au poids total de la composition.

Cette composition peut être utilisée comme, ou pour la fabrication d'une, composition démaquillante.

L'un des intérêts de cette composition est d'assurer un démaquillage à la fois efficace et non irritant. Les compositions d'esters conformes à l'invention ont en effet montré un parfait équilibre entre effet solvant d'une part et innocuité d'autre part.

En outre, grâce à la composition de l'invention, il y a un gain peu commun en fraîcheur et agrément d'utilisation par rapport aux démaquillants classiques.

De plus, le démaquillage peut être réalisé sans étape de rinçage, ce qui est recherché par les personnes utilisatrices qui apprécient, de nos jours, de gagner du temps.

Un autre avantage de la composition de l'invention est qu'elle est adaptée à l'élimination de tout type de produits de maquillage, y compris les maquillages "waterproof" pour les yeux ou les produits de maquillage aux textures riches en gras, tels que des fonds de teint, poudres, rouges-à-lèvres, servant notamment au maquillage des acteurs.

En outre, elle trouve une application très intéressante dans les pays où il fait chaud et où des démaquillants trop riches donnant une sensation de lourdeur sont mal supportés.

L'invention va maintenant être décrite plus en détails.

L'ester rentrant dans la composition de l'invention est de préférence présent à une concentration variant de 1% à 5% en poids par rapport au poids total de la composition.

Cet ester représente de préférence de 40% à 100%, et de manière encore plus préférée de 70% à 100%, du poids total de la phase grasse présente dans la composition.

Ledit ester est de préférence obtenu à partir d'un alcool à chaîne droite, ou ramifiée, ayant un nombre d'atomes de carbone choisi de 1 à 17 et d'un acide gras à chaîne droite, ou ramifiée, ayant un nombre d'atomes de carbone choisi de 3 à 18. Il comporte avantageusement de 12 à 17 atomes de carbone.

Cet ester est de préférence un mono- ou un di-ester.

De manière préférée, cet ester est choisi dans le groupe des esters qui ne comportent aucune insaturation et/ou aucun groupement éther ou hydroxyle.

De façon encore plus avantageuse, ledit ester est un ester saturé qui ne renferme aucun groupement éther ni hydroxyle.

Ainsi, l'ester de la composition conforme à l'invention peut être notamment choisi dans le groupe constitué par l'adipate de di-isopropyle, l'hexanoate d'éthyl-2 hexyle, le laurate d'éthyle, le myristate de méthyle, le néopentanoate d'isodécyle, le myristate d'éthyle, le propionate de myristyle, l'éthyl-2 hexanoate d'éthyle-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle.

Il va bien entendu de soi que les compositions selon l'invention peuvent contenir un ou plusieurs esters tels qu'ils ont été définis ci-avant.

La composition de l'invention peut se présenter, selon une première variante, sous la forme d'une lotion biphasique à interface nette entre la phase aqueuse et la phase grasse.

La composition selon l'invention peut comprendre, en outre, selon une autre variante, un agent permettant une mise en suspension de la phase grasse par action électrostérique.

Dans ce cas, cet agent de mise en suspension est, de préférence, un copolymère d'acrylates d'alkyle en C₁₀-C₃₀ et d'acide acrylique ou méthacrylique ou de leur ester.

De tels copolymères sont par exemple vendus par la Société GOODRICH sous la dénomination Pemulen TR1, Pemulen TR2, Carbopol 1342.

Ledit agent de mise en suspension de la phase grasse peut être présent à une teneur comprise entre 0,05% et 2% en poids par rapport au poids total de la composition. On peut ainsi obtenir, selon les teneurs choisies, un lait ou une crème.

La composition peut aussi comprendre, selon une autre variante, un agent de mise en dispersion de la phase grasse, qui agit par diminution de la tension interfaciale. Dans ce cas, cet agent peut être formé d'un système émulsionnant ou d'un système vésiculaire à base de vésicules, éventuellement de taille nanométrique, constituées de lipides ioniques (liposomes) ou non ioniques.

Les systèmes émulsionnants sont bien connus de l'homme du métier. lls peuvent être constitués par exemple du glycéryl stéarate/PEG 100 stéarate (CTFA) et d'alcool cétylique.

Lorsque l'agent de dispersion de la phase grasse est un système vésiculaire, il est formé de préférence du mélange lécithine hydrogénée/stérols de soja oxyéthylénés (5 moles d'oxyde d'éthylène).

D'autres systèmes vésiculaires pour disperser la phase grasse de la composition de l'invention peuvent également convenir. Pour préparer de tels systèmes, on peut avantageusement suivre l'enseignement des documents FR-A-2315991 ou FR-A-2543018.

Ledit agent de mise en dispersion de la phase grasse peut être présent à une teneur pondérale choisie entre 0,05% et 2% par rapport à l'ensemble de la composition.

La composition de l'invention peut comprendre, en outre, un agent pour augmenter sa viscosité, et ceci afin d'obtenir des démaquillants présentant des textures plus ou moins gélifiées.

Dans ce cas, cet agent est un gélifiant, notamment de la phase aqueuse, et peut être choisi dans le groupe constitué par :
- les polysaccharides tels que les dérivés cellulosiques (carboxyméthylcellulose, hydroxyéthylcellulose, hydroxypropylméthylcellulose), les gommes naturelles telles que les gommes de xanthane (vendues par la Société KELCO sous la dénomination Keltrol T), de guar, de caroube, les scléroglucanes, les dérivés de chitine ou de chitosane, les carraghénanes (vendues par la Société SANOFI sous la dénomination Satiagel K80),
- les dérivés polycarboxyvinyliques du type Carbomer (vendues par la Société GOODRICH sous les dénominations Carbopol, 940, 951, 980, ou par la Société 3V-SIGMA sous la dénomination Synthalen K ou Synthalen L).

L'agent gélifiant peut être présent à une teneur qui varie de 0,1% à 2% du poids total de la composition.

La composition de l'invention peut également inclure un agent actif, cosmétiquement acceptable.

De préférence, cet agent est choisi parmi les hydratants, tels que notamment la glycérine, le butylène glycol, les anti-inflammatoires comme l'allantoïne, le bisabolol, les anti-radicaux libres comme la vitamine E ou ses dérivés, les apaisants tels que l'eau de bleuet, l'extrait d'iris, ou tout autre extrait naturel hydrosoluble de végétaux.

Dans l'application démaquillante, la composition de l'invention présente un pH qui respecte la peau, généralement compris entre 5 et 8, de préférence un pH de 6 à 7,5.

A cet effet, une composition préférée selon l'invention comprend (% pondéraux) : de 1% à 10% d'au moins un ester dont le nombre total d'atomes de carbone est choisi de 12 à 20, de 0,05% à 1% d'un agent de mise en suspension, une quantité suffisante d'une base neutralisante pour obtenir un pH de 6,5 et de l'eau pour avoir 100%.

Une autre composition plus particulièrement préférée comprend (% pondéraux): 8% d'hexanoate d'éthyl-2 hexyle, 0,1% d'un copolymère acide acrylique/méthacrylate de stéaryle polymérisé dans un mélange d'acétate d'éthyle/cyclohexane, 0,1% de triéthanolamine, 0,1% de bisabolol (CTFA), 1% d'eau de bleuet, une quantité suffisante de soude pour obtenir un pH de 6,5 et d'eau pour avoir 100 %.

D'autres caractéristiques et avantages des compositions d'ester(s) de l'invention pourront apparaître dans les exemples qui suivent, lesquels sont donnés à titre purement illustratif et nullement limitatif.

Ces exemples seront précédés par une brève description de la technique de contrôle qui a été utilisée pour apprécier le pouvoir démaquillant des compositions.

Le pouvoir démaquillant a été contrôlé à l'aide de la technique dite du "robot démaquilleur" :
l'appareil se compose d'une plaque et d'un bras muni d'un poids exerçant sur la plaque une pression de 100g/cm² et équipé à l'une de ses extrémités d'un coton qui glisse sur la plaque.

On dépose sur la plaque une fine couche d'un mascara waterproof noir commercialisé sous le nom d'Aquacils par la Société Lancôme.
Cette plaque est mise à sécher pendant 4 Heures. Au bout de quatre heures, on réalise 9 passages simples sans retour, d'un coton imbibé de 40 gouttes de composition, le coton étant changé après chaque passage.

On évalue visuellement la quantité de mascara restante sur la plaque après ces neuf passages. L'absence totale de mascara sur la plaque constitue, selon ce test, un démaquillage parfait.

### Exemple 1 :

Les esters sélectionnés pour cet exemple ont été formulés dans la composition suivante (% pondéraux) :

| | |
|---|---|
| -Ester | 5% (invention) |
| ou | 30% (art antérieur) |
| -Agent de mise en suspension (Pemulen TR1 (GOODRICH)) | 0,1% |
| -Triéthanolamine . | 0,1% |
| -Eau | qsp 100% |

Après mise en oeuvre du test défini ci-avant, on a constaté qu'une composition à 5% en ester C₁₂ (adipate de di-isopropyle) et qu'une composition à 5% en ester C₁₄ (hexanoate d'éthyl-2 hexyle) conformes à l'invention possèdent toute deux un pouvoir démaquillant supérieur à celui d'une composition à 30% en ester C₂₇ (myristate d'isotridécyle) de l'art antérieur.

On va maintenant donner d'autres exemples de formulations rentrant dans le cadre de l'invention. Les quantités sont exprimées en grammes.

### Exemple 2 : Lotion démaquillante biphasique

| | |
|---|---|
| - Hexanoate d'éthyl-2 hexyle | 8 |
| - Bisabolol (CTFA) | 0,1 |
| - Chlorure de sodium | 0,15 |
| - Phosphate monopotassique | 0,1 |
| - Phosphate dipotassique | 0,3 |
| - Eau de bleuet | 1 |
| - Parahydroxybenzoate de méthyle | 0,2 |
| - Imidazolidinylurée | 0,15 |
| - Hydroxyde de sodium | qsp pH 6,5-7 |
| - Eau | qsp 100 |

Cette lotion biphasique permet un démaquillage efficace et frais du visage et des yeux, même pour un maquillage waterproof.

### Exemple 3 : Lait démaquillant

| | |
|---|---|
| - Myristate d'isopropyle | 5 |
| - Copolymère acide acrylique/méthacrylate de stéaryle polymérisé dans un mélange d'acétate d'éthyle/cyclohexane | 0,1 |
| - Gomme de xanthane | 0,1 |
| - Allantoïne | 0,1 |
| - Imidazolidinylurée | 0,15 |
| - Parahydroxybenzoate de méthyle | 0,2 |
| - Triethanolamine | 0,1 |
| - Eau | qsp 100 |

Ce lait très peu gras et réalisé sans tensioactif permet un démaquillage efficace sans provoquer de sensation de tiraillement ou d'irritation. Il convient spécialement aux peaux dites sensibles.

### Exemple 4 : Crème démaquillante

| | |
|---|---|
| - Propionate de myristyle | 10 |
| - Glyceryl stearate/PEG 100 stearate (CTFA) | 1,5 |
| - Alcool cétylique | 1 |
| - Diéthanolamide de coprah étété | 1 |
| - Extrait d'iris | 0,05 |
| - Glycérine | 3 |
| - Phénoxy-2 éthanol | 0,05 |
| - Carbomer (CTFA) (vendu par 3V-SIGMA sous la dénomination Synthalen L) | 0,6 |
| - Hydroxyde de sodium | 0,168 |
| - parahydroxybenzoate de méthyle | 0,15 |
| - Imidazolidinylurée | 0,05 |
| - Eau | qsp 100 |

Cette crème démaquillante convient pour tout type de maquillage. Elle est plutôt adaptée aux peaux sèches tout en apportant un maximum de fraîcheur.

### Exemple 5 : Gel-crème démaquillant

| | |
|---|---|
| - Palmitate d'éthyle | 2 |
| - Lécithine hydrogénée | 0,66 |
| - Stérols de soja oxyéthylénés 5 0E | 0,44 |
| - Cyclométhicone (CTFA) | 0,5 |
| - Carbomer (voir exemple 3) | 0,45 |
| - D-Galactopyrannose sulfate | 0,4 |
| - Hydroxyde de sodium | 0,13 |
| - Butylène glycol | 7 |
| - parahydroxybenzoate de méthyle et de propyle | 0,1 |
| - Imidazolidinylurée | 0,25 |
| - Eau | qsp eau |

Ce gel liposomé assure un démaquillage très frais, sans sensation de gras.

Après application et démaquillage du visage et/ou des yeux à l'aide des compositions 2 à 5 illustrées ci-dessus, un rinçage n'est pas indispensable. Une sensation de confort et de fraîcheur peu commune a été ressentie par un panel de 12 personnes.

Par ailleurs, toutes ces compositions sont restées stables au cours du temps.

## Revendications

1. Composition formée d'une phase aqueuse et d'une phase grasse, ladite phase grasse comprenant au moins un ester, caractérisée en ce que ledit ester a un nombre total d'atomes de carbone allant de 12 à 20, en ce qu'il est présent à une teneur pondérale comprise entre 0,1% et 10% du poids total de la composition et en ce que la phase grasse représente moins de 20 % en poids par rapport au poids total de la composition.

2. Composition selon la revendication 1, caractérisée en ce que l'ester est présent à raison de 1% à 5% du poids total de la composition.

3. Composition selon l'une quelconque des revendications précédentes, caractérisée en ce que l'ester représente de 40% à 100% du poids total de la phase grasse.

4. Composition selon la revendication 3, caractérisée en ce que l'ester représente de 70% à 100% du poids total de la phase grasse.

5. Composition selon l'une quelconque des revendications précédentes, caractérisée en ce que la phase grasse représente de 3 à 15% en poids par rapport au poids total de la composition.

6. Composition selon l'une quelconque des revendications précédentes, caractérisée en ce que l'ester a un nombre total d'atomes de carbone allant de 12 à 17.

7. Composition selon l'une quelconque des revendications précédentes, caractérisée en ce que l'ester est obtenu à partir d'un alcool à chaîne droite, ou ramifiée, ayant un nombre d'atomes de carbone choisi de 1 à 17 et d'un acide gras à chaîne droite, ou ramifiée, ayant un nombre d'atomes de carbone choisi de 3 à 18.

8. Composition selon l'une quelconque des revendications précédentes, caractérisée en ce que l'ester ne comporte aucune insaturation.

9. Composition selon l'une quelconque des revendications précédentes, caractérisée en ce que l'ester ne comporte aucun groupement éther ou hydroxyle.

10. Composition selon l'une quelconque des revendications précédentes, caractérisée en ce que l'ester ne comporte aucune insaturation, ni aucun groupement éther ni aucun groupement hydroxyle.

11. Composition selon l'une quelconque des revendications précédentes, caractérisée en ce que l'ester est choisi seul ou en mélange dans le groupe constitué par l'adipate de di-isopropyle, l'hexanoate d'éthyl-2 hexyle, le laurate d'éthyle, le myristate de méthyle, le néopentanoate d'isodécyle, le myristate d'éthyle, le propionate de myristyle, I'éthyl-2 hexanoate d'éthyle-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle.

12. Composition selon l'une quelconque des revendications précédentes, caractérisée en ce qu'elle comprend un agent de mise en suspension de la phase grasse par action électrostérique, ou un agent de mise en dispersion de la phase grasse par diminution de la tension interfaciale.

13. Composition selon la revendication 12, caractérisée en ce que ledit agent de mise en suspension est un copolymère d'acrylates d'alkyle en C₁₀-C₃₀ et d'acide (metha) acrylique ou de ses esters.

14. Composition selon la revendication 13, caractérisée en ce que le copolymère est un copolymère d'acide acrylique/methacrylate de stéaryle polymérisé dans le mélange acétate d'éthyle/cyclohexane.

15. Composition selon l'une quelconque des revendications 12 à 14, caractérisée en ce que ledit agent de mise en suspension est présent à une teneur comprise entre 0,05% et 2% du poids total de la composition.

16. Composition selon la revendication 12, caractérisée en ce que ledit agent de mise en dispersion de la phase grasse est formé d'un système émulsionnant ou d'un système vésiculaire à base de vésicules, éventuellement de taille nanométrique, constituées de lipides ioniques (liposomes) ou non ioniques.

17. Composition selon la revendication 16, caractérisée en ce que le système émulsionnant est constitué du glycéryl stéarate/PEG 100 stéarate (CTFA) et d'alcool cétylique.

18. Composition selon la revendication 16, caractérisée en ce que le système vésiculaire est formé du mélange lécithine hydrogénée/stérols de soja oxyéthylénés (5 moles d'oxyde d'éthylène).

19. Composition selon la revendication 12 ou 16, caractérisée en ce que l'agent de mise en dispersion de la phase grasse est présent à une teneur choisie entre 0,05% et 2% du poids total de la composition.

20. Composition selon l'une quelconque des revendications précédentes, caractérisée en ce qu'elle comprend un agent pour augmenter sa viscosité.

21. Composition selon la revendication 20, caractérisée en ce que ledit agent est choisi dans le groupe constitué par :
- les polysaccharides tels que les dérivés cellulosiques (carboxyméthylcellulose, hydroxyéthylcellulose, hydroxypropylméthylcellulose), les gommes naturelles telles que les gommes de xanthane, de guar, de caroube, les scléroglucanes, les dérivés de chitine ou de chitosane, les carraghénanes.
- les dérivés polycarboxyvinyliques du type Carbomer.

22. Composition selon la revendication 20 ou 21, caractérisée en ce que l'agent pour augmenter la viscosité est présent à une teneur comprise entre 0,1% et 2% du poids total de la composition.

23. Composition selon l'une quelconque des revendications précédentes, caractérisée en ce qu'elle comprend un agent actif, cosmétiquement acceptable.

24. Composition selon la revendication 23, caractérisée en ce que l'actif est choisi, seul ou en mélange, dans le groupe constitué par le bisabolol, l'eau de bleuet, I'allantoïne, la glycérine, l'extrait d'iris, le butylène glycol, la vitamine E ou ses dérivés.

25. Composition selon l'une quelconque des revendications précédentes, caractérisée en ce qu'elle se présente sous la forme d'une lotion biphasique, d'un lait sans émulsionnant, d'une émulsion de consistance crémeuse, d'un gel de consistance crémeuse résultant de la dispersion de la phase grasse de ladite composition par des vésicules lipidiques ioniques hydratées (liposomes).

26. Composition selon l'une quelconque des revendications précédentes, caractérisée en ce qu'il s'agit d'une composition pour le démaquillage.

27. Utilisation d'une composition selon l'une quelconque des revendications précédentes comme, ou pour la fabrication d'une, composition démaquillante.

28. Procédé de démaquillage, caractérisé en ce qu'on applique sur de la peau ou sur des yeux maquillés une composition selon l'une quelconque des revendications 1 à 26.

29. Procédé selon la revendication 28, caractérisé en ce qu'il ne comporte pas d'étape de rinçage.

## Claims

1. Composition formed of an aqueous phase and of a fatty phase, the said fatty phase comprising at least one ester, characterized in that the said ester has a total number of carbon atoms ranging from 12 to 20, in that it is present at a content by weight of between 0.1 % and 10 % of the total weight of the composition and in that the fatty phase represents less than 20 % by weight relative to the total weight of the composition.

2. Composition according to Claim 1, characterized in that the ester is present in an amount of 1 % to 5 % of the total weight of the composition.

3. Composition according to either of the preceding claims, characterized in that the ester represents from 40 % to 100 % of the total weight of the fatty phase.

4. Composition according to Claim 3, characterized in that the ester represents from 70 % to 100 % of the total weight of the fatty phase.

5. Composition according to any one of the preceding claims, characterized in that the fatty phase represents from 3 to 15 % by weight relative to the total weight of the composition.

6. Composition according to any one of the preceding claims, characterized in that the ester has a total number of carbon atoms ranging from 12 to 17.

7. Composition according to any one of the preceding claims, characterized in that the ester is obtained from a straight- or branched-chain alcohol having a number of carbon atoms chosen from 1 to 17 and a straight- or branched-chain fatty acid having a number of carbon atoms chosen from 3 to 18.

8. Composition according to any one of the preceding claims, characterized in that the ester contains no unsaturation.

9. Composition according to any one of the preceding claims, characterized in that the ester contains no ether or hydroxyl group.

10. Composition according to any one of the preceding claims, characterized in that the ester contains neither any unsaturation nor any ether group nor any hydroxyl group.

11. Composition according to any one of the preceding claims, characterized in that the ester is chosen, alone or as a mixture, from the group consisting of diisopropyl adipate, 2-ethylhexyl hexanoate, ethyl laurate, methyl myristate, isodecyl neopentanoate, ethyl myristate, myristyl propionate, 2-ethylhexyl 2-ethylhexanoate, 2-ethylhexyl octanoate, 2-ethylhexyl caprate/caprylate, methyl palmitate, butyl myristate, isobutyl myristate, ethyl palmitate, isohexyl laurate and hexyl laurate.

12. Composition according to any one of the preceding claims, characterized in that it comprises an agent for suspending the fatty phase by electrosteric action, or an agent for dispersing the fatty phase by decreasing the interface tension.

13. Composition according to Claim 12, characterized in that the said suspension agent is a copolymer of C₁₀-C₃₀ alkyl acrylates and (meth)acrylic acid or its esters.

14. Composition according to Claim 13, characterized in that the copolymer is a polymerized acrylic acid/stearyl methacrylate copolymer in an ethyl acetate/cyclohexane mixture.

15. Composition according to one of Claims 12 to 14, characterized in that the said suspension agent is present at a content between 0.05 % and 2 % of the total weight of the composition.

16. Composition according to Claim 12, characterized in that the said agent for dispersing the fatty phase is formed of an emulsifying system or of a vesicle system based on vesicles, possibly of nanometre size, consisting of ionic lipids (liposomes) or nonionic lipids.

17. Composition according to Claim 16, characterized in that the emulsifying system consists of glyceryl stearate/PEG 100 stearate (CTFA) and cetyl alcohol.

18. Composition according to Claim 16, characterized in that the vesicle system is formed of the hydrogenated lecithin/oxyethylenated soya sterols (5 mol of ethylene oxide) mixture.

19. Composition according to Claim 12 or 16, characterized in that the agent for dispersing the fatty phase is present at a content chosen between 0.05 % and 2 % of the total weight of the composition.

20. Composition according to any one of the preceding claims, characterized in that it comprises an agent for increasing its viscosity.

21. Composition according to Claim 20, characterized in that the said agent is chosen from the group consisting of:
- polysaccharides such as cellulose derivatives (carboxymethyl cellulose, hydroxyethyl cellulose and hydroxypropylmethyl cellulose), natural gums such as xanthan gum, guar gum and carob gum, scleroglucans, derivatives of chitin or of chitosan, and carrageenans,
- polycarboxyvinyl derivatives of the Carbomer type.

22. Composition according to Claim 20 or 21, characterized in that the agent for increasing the viscosity is present at a content between 0.1 % and 2 % of the total weight of the composition.

23. Composition according to any one of the preceding claims, characterized in that it comprises a cosmetically acceptable active agent.

24. Composition according to Claim 23, characterized in that the active agent is chosen, alone or as a mixture, from the group consisting of bisabolol, cornflower water, allantoin, glycerol, extract of iris, butylene glycol and vitamin E or its derivatives.

25. Composition according to any one of the preceding claims, characterized in that it is provided in the form of a two-phase lotion, an emulsifying agent-free milk, an emulsion of creamy consistency or a gel of creamy consistency resulting from the dispersion of the fatty phase of the said composition by hydrated ionic lipid vesicles (liposomes).

26. Composition according to any one of the preceding claims, characterized in that it is a make-up removal composition.

27. Use of a composition according to any one of the preceding claims as, or for the production of, a make-up removal composition.

28. Make-up removing process, characterized in that a composition according to any one of Claims 1 to 26 is applied to made-up skin or eyes.

29. Process according to Claim 28, characterized in that it does not include a rinsing step.

## Patentansprüche

1. Zusammensetzungen aus einer wäßrigen Phase und einer Fettphase, wobei die Fettphase mindestens einen Ester enthält,
dadurch gekennzeichnet, daß
der Ester eine Gesamtzahl von Kohlenstoffatomen im Bereich von 12 bis 20 aufweist, der Ester in einem Gewichtsanteil von 0,1 bis 10 % des Gesamtgewichts der Zusammensetzung vorliegt und die Fettphase weniger als 20 Gew.-%, bezogen auf das Gesamtgewicht der Zusammensetzung, ausmacht.

2. Zusammensetzungen nach Anspruch 1, dadurch gekennzeichnet, daß der Ester in einem Anteil von 1 bis 5 % des Gesamtgewichts der Zusammensetzung vorliegt.

3. Zusammensetzungen nach einem der vorhergehenden Ansprüche, dadurch gekennzeichnet, daß der Ester 40 bis 100 % des Gesamtgewichts der Fettphase ausmacht.

4. Zusammensetzung nach Anspruch 3, dadurch gekennzeichnet, daß der Ester 70 bis 100 % des Gesamtgewichts der Fettphase ausmacht.

5. Zusammensetzungen nach einem der vorhergehenden Ansprüche, dadurch gekennzeichnet, daß die Fettphase 3 bis 15 Gew.-%, bezogen auf das Gesamtgewicht der Zusammensetzung, ausmacht.

6. Zusammensetzungen nach einem der vorhergehenden Ansprüche, dadurch gekennzeichnet, daß der Ester eine Gesamtzahl von Kohlenstoffatomen im Bereich von 12 bis 17 aufweist.

7. Zusammensetzungen nach einem der vorhergehenden Ansprüche, dadurch gekennzeichnet, daß der Ester aus einem geradkettigen oder verzweigten Alkohol mit einer Anzahl von Kohlenstoffatomen im Bereich von 1 bis 17 und einer geradkettigen oder verzweigten Fettsäure mit einer Anzahl von Kohlenstoffatomen im Bereich von 3 bis 18 hergestellt ist.

8. Zusammensetzungen nach einem der vorhergehenden Ansprüche, dadurch gekennzeichnet, daß der Ester keine ungesättigten Bindungen aufweist.

9. Zusammensetzungen nach einem der vorhergehenden Ansprüche, dadurch gekennzeichnet, daß der Ester weder Ethergruppen noch Hydroxygruppen aufweist.

10. Zusammensetzungen nach einem der vorhergehenden Ansprüche, dadurch gekennzeichnet, daß der Ester weder eine ungesättigte Bindung noch eine Ethergruppe noch eine Hydroxygruppe aufweist.

11. Zusammensetzungen nach einem der vorhergehenden Ansprüche, dadurch gekennzeichnet, daß der Ester unter Diisopropyladipat, 2-Ethylhexylhexanoat, Ethyllaurat, Methylmyristat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat oder deren Gemischen ausgewählt ist.

12. Zusammensetzungen nach einem der vorhergehenden Ansprüche, dadurch gekennzeichnet, daß sie ein Mittel zur Suspendierung der Fettphase mit elektrosterischer Wirkung oder ein Mittel zur Dispergierung der Fettphase durch Verminderung der Grenzflächenspannung enthält.

13. Zusammensetzungen nach Anspruch 12, dadurch gekennzeichnet, daß das Suspendiermittel ein Copolymer von C₁₀₋₃₀-Alkylacrylaten und (Meth)acrylsäure oder deren Estern ist.

14. Zusammensetzungen nach Anspruch 13, dadurch gekennzeichnet, daß das Copolymer ein Copolymer von Acrylsäure und Stearylmethacrylat ist, das in einem Ethylacetat-Cyclohexan-Gemisch polymerisiert wurde.

15. Zusammensetzungen nach einem der Ansprüche 12 bis 14, dadurch gekennzeichnet, daß das Suspendiermittel in einem Anteil von 0,05 bis 2 % des Gesamtgewichts der Zusammensetzung vorliegt.

16. Zusammensetzungen nach Anspruch 12, dadurch gekennzeichnet, daß das Mittel zu Dispergierung der Fettphase aus einem Emulgatorsystem oder aus einem Vesikelsystem auf der Basis von Vesikeln gegebenenfalls einer Größe im Nanometerbereich, die aus ionischen Lipiden (Liposomen) oder nichtionischen Lipiden gebildet werden, besteht.

17. Zusammensetzungen nach Anspruch 16, dadurch gekennzeichnet, daß das Emulgatorsystem aus Glycerylstearat/PEG 100-Stearat (CTFA) und Cetylalkohol besteht.

18. Zusammensetzungen nach Anspruch 16, dadurch gekennzeichnet, daß das Vesikelsystem aus einem Gemisch von hydriertem Lecithin und ethoxylierten (5 mol Ethylenoxid) Sojasterinen gebildet wird.

19. Zusammensetzungen nach Anspruch 12 oder 16, dadurch gekennzeichnet, daß das Mittel zur Dispergierung der Fettphase in einem Anteil von 0,05 bis 2 % des Gesamtgewichts der Zusammensetzung vorliegt.

20. Zusammensetzungen nach einem der vorhergehenden Ansprüche, dadurch gekennzeichnet, daß sie ein Mittel zur Erhöhung der Viskosität enthalten.

21. Zusammensetzung nach Anspruch 20, dadurch gekennzeichnet, daß dieses Mittel ausgewählt ist unter:
- den Polysacchariden, wie den Cellulosederivaten (Carboxymethylcellulose, Hydroxyethylcellulose, Hydroxypropylmethylcellulose), den natürlichen Gummen, wie Xanthangummi, Guargummi, Johannisbrotkernmehl, den Scleroglucanan, Chitin- oder Chitosanderivaten und Carrageenanen; und
- den Polycarboxyvinylderivaten vom Carbomertyp.

22. Zusammensetzungen nach Anspruch 20 oder 21, dadurch gekennzeichnet, daß das Mittel zur Erhöhung der Viskosität in einem Anteil von 0,1 bis 2 % des Gesamtgewichts der Zusammensetzung vorliegt.

23. Zusammensetzungen nach einem der vorhergehenden Ansprüche, dadurch gekennzeichnet, daß sie einen kosmetisch akzeptablen Wirkstoff enthalten.

24. Zusammensetzung nach Anspruch 23, dadurch gekennzeichnet, daß der Wirkstoff unter Bisabolol, Kornblumenwasser, Allantoin, Glycerin, Irisextrakt, Butylenglykol, Vitamin E oder seinen Derivaten und deren Gemischen ausgewählt ist.

25. Zusammensetzungen nach einem der vorhergehenden Ansprüche, dadurch gekennzeichnet, daß sie in Form einer zweiphasigen Lotion, in Form einer Milch ohne Emulgator, einer Emulsion von cremiger Konsistenz oder eines Gels von cremiger Konsistenz vorliegen, welche aus der Dispersion der Fettphase der Zusammensetzung durch hydratisierte ionische Lipidvesikel (Liposomen) resultieren.

26. Zusammensetzungen nach einem der vorhergehenden Ansprüche, dadurch gekennzeichnet, daß es sich um Zusammensetzungen zum Abschminken handelt.

27. Verwendung einer Zusammensetzung nach einem der vorhergehenden Ansprüche als Zusammensetzung zum Abschminken oder zu deren Herstellung.

28. Verfahren zum Abschminken, dadurch gekennzeichnet, daß auf die geschminkte Haut oder die geschminkten Augen eine Zusammensetzung nach einem der Ansprüche 1 bis 26 aufgetragen wird.

29. Verfahren nach Anspruch 28, dadurch gekennzeichnet, daß kein Spülschritt enthalten ist.
